# EUROPEAN PATENT APPLICATION

(11) **EP 4 817 628 A1**
(43) Date of publication of application: **07.10.2026**
(21) Application number: 24898231.6
(22) Date of filing: 29.11.2024
(51) Int. Cl.: A61K 39/395, A61K 9/08, A61K 47/26, A61K 47/20, A61K 47/14, A61K 9/00, A61P 17/00, A61P 11/00, A61P 11/02, A61P 29/00

(54) **STABLE LIQUID FORMULATION OF ANTI-IL-4R alpha ANTIBODY**

(30) Priority: 01.12.2023 KR 20230172608
(71) Applicant: Samsung Bioepis Co., Ltd., Yeonsu-gu Incheon 21987 (KR)
(72) Inventor: PARK, Jin Hye, Incheon 21982 (KR); KIM, Han Soo, Incheon 22001 (KR); BOCK, Sung Je, Incheon 21982 (KR); KIM, In Ae, Seoul 05090 (KR); LEE, Hun Joo, Incheon 21996 (KR); CHANG, Sung Keun, Incheon 22003 (KR)
(74) Representative: Boult Wade Tennant LLP
(86) International application number: PCT/KR2024/019353
(87) International publication number: WO 2025/116637

(57) **Abstract**

The present disclosure relates to a liquid formulation comprising an anti-IL-4Rα antibody, a buffer, a saccharide, and methionine, and having a pH of 4.9 to 5.5, a device comprising the same, and a use thereof for treating an IL-4Rα-related condition. The liquid formulation may secure the stability of the antibody while simultaneously improving the viscosity, and thus can be usefully utilized as a medicament for treating an IL-4Rα-related condition.

## Description

### Technical Field

The present disclosure relates to a stable liquid formulation of an anti-IL-4Rα antibody, a device comprising the same, and a use thereof for treating an IL-4Rα-related condition.

### Background Art

Antibody drugs have a large molecular weight and complex secondary/higher-order structures compared to general protein drugs, which may lead to physicochemical instability. For this reason, the development of an optimal formulation that guarantees the quality and stability of antibody drugs throughout the entire process of manufacture, storage, and administration to a patient is required. Protein instability can arise from various external factors such as temperature, light, and chemicals, which may reduce activity and decrease drug efficacy, or cause immunogenicity upon administration to the human body. Therefore, it is important to address such instability of antibody drugs to maintain optimal quality until they are administered to a patient. To achieve optimal quality, methods such as changing the buffer solution, evaluating an optimal pH, and adding a stabilizer are used. Because each antibody protein has different material properties, and the optimal combinations of a buffer solution, a pH, a stabilizer, and the like may vary, the development of a formulation suitable for the target material is required.

For example, dupilumab, an anti-IL-4Rα antibody, is manufactured and sold under the trade name Dupixent^{®}. Dupixent 200 mg is a 1.14 mL liquid preparation composed of 200 mg of an anti-IL-4Rα IgG, 1.2 mg of sodium acetate, 3.5 mg of histidine, 12 mg of arginine, 57 mg of sucrose, and 2.3 mg of polysorbate 80. Dupixent 300 mg is a 2.00 mL liquid preparation composed of 300 mg of an anti-IL-4Rα IgG, 2.0 mg of sodium acetate, 6.2 mg of histidine, 10.5 mg of arginine, 100 mg of sucrose, and 4 mg of polysorbate 80. To stabilize liquid preparations containing various antibody proteins such as dupilumab, various conditions are being investigated and applied, including the types and concentrations of components such as saccharides, amino acids, buffers, and surfactants , as well as the application of specific pH values, as exemplified by the composition of the Dupixent product.

In addition to securing the stability described above, and in consideration of patient convenience during injection, there is a need to develop high-concentration preparations that offer increased therapeutic activity while reducing the number of administrations through small-volume, high-dose injections. In the formulation development of high-concentration preparations, increasing the protein concentration introduces limitations, such as aggregation-induced protein destabilization and impaired injectability resulting from elevated viscosity. Therefore, it is critical to develop a preparation that improves viscosity while ensuring protein stability. Although saccharides are commonly used as stabilizers for proteins, preparations containing such stabilizers tend to exhibit accelerated viscosity increases at higher protein concentrations, which limits the ability to simultaneously secure protein stability and improve viscosity. To address the foregoing, a preparation can be developed by including a viscosity reducing agent, which inhibits protein aggregation to simultaneously secure protein stability and improve viscosity.

However, verification of the developed preparation requires a long period of time, since key quality attributes must be evaluated under storage temperature conditions through the expiration date to confirm whether viscosity and protein stability are improved up to administration to a patient. To efficiently evaluate whether protein stability is maintained and to identify the optimal preparation in a short period of time, the optimal preparation combination can be selected by analyzing novel quality indicators after exposing the protein to factors that cause structural and physicochemical denaturation, such as heat (temperature) and chemical substances.

### Disclosure of Invention

### Technical Problem

The present disclosure relates to a stable liquid formulation of an anti-IL-4Rα antibody such as dupilumab. Specifically, the present disclosure provides a liquid formulation capable of securing the stability of an anti-IL-4Rα antibody such as dupilumab throughout the entire process of manufacture, storage, and administration to a patient, while simultaneously improving the viscosity of the formulation. Because the liquid formulation exhibits improved stability and viscosity compared to a commercialized formulation of an anti-IL-4Rα antibody such as dupilumab (e.g., Dupixent), the liquid formulation is applicable to high-concentration preparations, and thus patient convenience may be increased.

One aspect is to provide a liquid formulation comprising an anti-IL-4Rα antibody, a buffer, a saccharide, and methionine, and having a pH of about 4.9 to about 5.5.

Another aspect is to provide a device comprising the liquid formulation.

Another aspect is to provide a method of treating an IL-4Rα-related condition, comprising administering the liquid formulation to a subject in need thereof.

Another aspect is to provide a use of the liquid formulation in the manufacture of a medicament for treating an IL-4Rα-related condition.

### Solution to Problem

Unless otherwise defined, all technical terms used herein have the same meaning as commonly understood by a person of ordinary skill in the art to which the present disclosure pertains. In addition, although preferred methods or samples are described herein, those similar or equivalent thereto are also included within the scope of the present disclosure. Furthermore, the numerical values described herein are considered to include the meaning of "about" even when not explicitly stated. The contents of all publications cited as references herein are incorporated herein by reference in their entirety.

As used herein, the term "about" or "approximately" may generally be interpreted to include values or ranges within±10 %, within±5 %, within±4 %, within±3 %, within±2 %, or within±1 % of a given value or range.

One aspect provides a stable liquid formulation of an anti-IL-4Rα antibody, and specifically, provides a liquid formulation comprising:
an anti-IL-4Rα antibody;
a buffer;
a saccharide; and
methionine, and
having a pH of about 4.9 to about 5.5.

### (1) Antibody

As used herein, the term "antibody" may be interpreted to include a full-length antibody or an antigen-binding fragment thereof. The antibody includes a monoclonal antibody, a polyclonal antibody, a humanized antibody, a human antibody, and a chimeric antibody.

The term "antigen-binding fragment" refers to a fragment including an antigen-binding site of an antibody. For example, the antigen-binding fragment includes, but is not limited to, a Fab fragment, an F(ab')₂ fragment, an Fc fragment, or an scFv fragment.

As used herein, the antibody may be an anti-IL-4Rα antibody. The anti-IL-4Rα antibody may refer to any antibody that binds to the interleukin-4 receptor alpha chain (IL-4Rα). The anti-IL-4Rα antibody can inhibit IL-4 and IL-13 signaling.

The anti-IL-4Rα antibody may be dupilumab (CAS No. 1190264-60-8). Therefore, the liquid formulation may be a stable liquid formulation of dupilumab. Dupilumab is a fully human monoclonal antibody that binds to IL-4Rα, and it belongs to IgG4. Dupilumab is sold under the trade name Dupixent^{®}. After being approved by the US Food and Drug Administration (FDA) as a treatment for atopic dermatitis, Dupixent is expanding its indications to various type 2 inflammatory diseases such as asthma, chronic rhinosinusitis with nasal polyposis (CRSwNP), and eosinophilic esophagitis (EoE). The sequence of dupilumab is known, and dupilumab may be produced by general methods known in the art. More detailed information on dupilumab may be easily obtained from known databases by a person skilled in the art.

As used herein, the term "dupilumab" may also be construed to encompass modified dupilumab in which the amino acid sequence is modified (by deletion, insertion, and/or substitution) and/or the glycosylation profile is modified, to an extent that it does not alter the polypeptide function.

The anti-IL-4Rα antibody may be included in the liquid formulation in a therapeutically effective amount.

The concentration of the anti-IL-4Rα antibody may be about 1 mg/mL to about 300 mg/mL, about 1 mg/mL to about 250 mg/mL, about 1 mg/mL to about 200 mg/mL, about 1 mg/mL to about 175 mg/mL, about 5 mg/mL to about 300 mg/mL, about 5 mg/mL to about 250 mg/mL, about 5 mg/mL to about 200 mg/mL, about 5 mg/mL to about 175 mg/mL, about 10 mg/mL to about 300 mg/mL, about 10 mg/mL to about 250 mg/mL, about 10 mg/mL to about 200 mg/mL, about 10 mg/mL to about 175 mg/mL, about 25 mg/mL to about 300 mg/mL, about 25 mg/mL to about 250 mg/mL, about 25 mg/mL to about 200 mg/mL, about 25 mg/mL to about 175 mg/mL, about 50 mg/mL to about 300 mg/mL, about 50 mg/mL to about 250 mg/mL, about 50 mg/mL to about 200 mg/mL, about 50 mg/mL to about 175 mg/mL, about 75 mg/mL to about 300 mg/mL, about 75 mg/mL to about 250 mg/mL, about 75 mg/mL to about 200 mg/mL, about 75 mg/mL to about 175 mg/mL, about 100 mg/mL to about 300 mg/mL, about 100 mg/mL to about 250 mg/mL, about 100 mg/mL to about 200 mg/mL, about 100 mg/mL to about 175 mg/mL, about 125 mg/mL to about 300 mg/mL, about 125 mg/mL to about 250 mg/mL, about 125 mg/mL to about 200 mg/mL, about 125 mg/mL to about 175 mg/mL, about 145 mg/mL to about 300 mg/mL, about 145 mg/mL to about 250 mg/mL, about 145 mg/mL to about 200 mg/mL, about 145 mg/mL to about 180 mg/mL, about 145 mg/mL to about 175 mg/mL, about 149 mg/mL to about 151 mg/mL, about 150 mg/mL to about 300 mg/mL, about 150 mg/mL to about 250 mg/mL, about 150 mg/mL to about 225 mg/mL, about 150 mg/mL to about 200 mg/mL, about 150 mg/mL to about 180 mg/mL, about 150 mg/mL to about 175 mg/mL, about 170 mg/mL to about 300 mg/mL, about 170 mg/mL to about 250 mg/mL, about 170 mg/mL to about 225 mg/mL, about 170 mg/mL to about 200 mg/mL, about 170 mg/mL to about 180 mg/mL, or about 174 mg/mL to about 176 mg/mL.

In an embodiment, the concentration of the anti-IL-4Rα antibody may be about 100 mg/mL to about 200 mg/mL.

In an embodiment, the concentration of the anti-IL-4Rα antibody may be about 125 mg/mL to about 200 mg/mL.

In an embodiment, the concentration of the anti-IL-4Rα antibody may be about 150 mg/mL to about 200 mg/mL.

In an embodiment, the concentration of the anti-IL-4Rα antibody may be about 150 mg/mL to about 175 mg/mL.

In an embodiment, the concentration of the anti-IL-4Rα antibody may be 135 mg/mL to 165 mg/mL.

In an embodiment, the concentration of the anti-IL-4Rα antibody may be 158 mg/mL to 192 mg/mL.

In a specific embodiment, the concentration of the anti-IL-4Rα antibody may be about 150 mg/mL.

In a specific embodiment, the concentration of the anti-IL-4Rα antibody may be about 175 mg/mL.

The concentration of the anti-IL-4Rα antibody may be a high concentration. Therefore, the liquid formulation may be a high-concentration liquid formulation of the anti-IL-4Rα antibody. The high concentration may mean 100 mg/mL or more or 150 mg/mL or more, for example, 100 mg/mL to 300 mg/mL or 150 mg/mL to 300 mg/mL, but is not limited thereto.

### (2) Buffer and pH

The liquid formulation according to one aspect comprises a buffer. The buffer may function to adjust the pH of the formulation. The pH of the liquid formulation may be maintained at a constant value or within a constant range by the buffer. Therefore, the buffer may serve to provide a constant value or a constant range of pH to the liquid formulation.

The buffer may be used without limitation in its type as long as it is applicable to biopharmaceuticals.

The buffer may comprise one or more selected from acetate, histidine, phosphate, citrate, succinate, malate, tartrate, carbonate, salts thereof, and hydrates thereof.

The term "salt" may refer to a pharmaceutically acceptable salt. The salt may comprise an inorganic acid salt, an organic acid salt, a metal salt, and the like of the compound. The inorganic acid salt may be a hydrochloride, hydrobromide, phosphate, sulfate, or disulfate. The organic acid salt may be a formate, acetate, propionate, lactate, oxalate, tartrate, malate, maleate, citrate, fumarate, besylate, camsylate, edisylate, trichloroacetate, trifluoroacetate, benzoate, gluconate, methanesulfonate, glycolate, succinate, 4-toluenesulfonate, galacturonate, embonate, glutamate, ethanesulfonate, benzenesulfonate, p-toluenesulfonate, or aspartate. The metal salt may be a calcium salt, a sodium salt, a magnesium salt, a strontium salt, or a potassium salt.

The term "hydrate" refers to a substance containing a water molecule within its molecule. The hydrate may be a monohydrate, a dihydrate, or a trihydrate.

The pH of the liquid formulation according to one aspect may be about 4.9 to about 5.5. The pH of the liquid formulation may be provided by the buffer. Alternatively, the pH of the liquid formulation may be adjusted by the addition of any acid (e.g., HCl) or base (e.g., NaOH).

Specifically, the pH of the formulation may be any range or any value selected within about 4.9 to about 5.5. For example, the pH may be about 4.9 to about 5.5, about 4.9 to about 5.4, about 4.9 to about 5.3, about 4.9 to about 5.2, about 5.0 to about 5.5, about 5.0 to about 5.4, about 5.0 to about 5.3, about 5.0 to about 5.2, about 5.1 to about 5.5, about 5.1 to about 5.4, or about 5.1 to about 5.3.

In an embodiment, the pH of the liquid formulation may be about 4.9, about 5.0, about 5.1, about 5.2, about 5.3, about 5.4, or about 5.5.

In an embodiment, the buffer may comprise one or more selected from acetate, histidine, salts thereof, and hydrates thereof.

In an embodiment, the buffer may comprise one or more selected from acetate, salts thereof, and hydrates thereof. In an embodiment, the buffer may comprise acetate. In an embodiment, the buffer may be acetate. In an embodiment, the buffer may comprise sodium acetate. In an embodiment, the buffer may be sodium acetate.

The concentration of the buffer may be any range or any value selected within about 0.1 mM to about 50 mM. For example, the concentration of the buffer may be about 0.1 mM to about 50 mM, about 0.1 mM to about 40 mM, about 0.1 mM to about 30 mM, about 1 mM to about 50 mM, about 1 mM to about 40 mM, about 1 mM to about 30 mM, about 5 mM to about 50 mM, about 5 mM to about 40 mM, about 5 mM to about 35 mM, about 5 mM to about 30 mM, about 10 mM to about 50 mM, about 10 mM to about 40 mM, about 10 mM to about 35 mM, about 10 mM to about 30 mM, about 15 mM to about 50 mM, about 15 mM to about 40 mM, about 15 mM to about 35 mM, about 15 mM to about 30 mM, about 20 mM to about 50 mM, about 20 mM to about 40 mM, about 20 mM to about 35 mM, about 20 mM to about 30 mM, about 25 mM to about 50 mM, about 25 mM to about 40 mM, about 25 mM to about 35 mM, about 27 mM to about 50 mM, about 27 mM to about 40 mM, about 27 mM to about 35 mM, or about 27 mM to about 33 mM. In a specific embodiment, the concentration of the buffer may be about 30 mM.

The concentration of the acetate may be any range or any value selected within about 0.1 mM to about 50 mM. For example, the concentration of the acetate may be about 0.1 mM to about 50 mM, about 0.1 mM to about 40 mM, about 0.1 mM to about 30 mM, about 1 mM to about 50 mM, about 1 mM to about 40 mM, about 1 mM to about 30 mM, about 5 mM to about 50 mM, about 5 mM to about 40 mM, about 5 mM to about 35 mM, about 5 mM to about 30 mM, about 10 mM to about 50 mM, about 10 mM to about 40 mM, about 10 mM to about 35 mM, about 10 mM to about 30 mM, about 15 mM to about 50 mM, about 15 mM to about 40 mM, about 15 mM to about 35 mM, about 15 mM to about 30 mM, about 20 mM to about 50 mM, about 20 mM to about 40 mM, about 20 mM to about 35 mM, about 20 mM to about 30 mM, about 25 mM to about 50 mM, about 25 mM to about 40 mM, about 25 mM to about 35 mM, about 27 mM to about 50 mM, about 27 mM to about 40 mM, about 27 mM to about 35 mM, or about 27 mM to about 33 mM.

In a specific embodiment, the concentration of the acetate may be about 30 mM.

### (3) Saccharide

The liquid formulation according to one aspect comprises a saccharide.

The saccharide may comprise one or more selected from a sugar and a sugar alcohol.

The sugar may be a monosaccharide, a disaccharide, an oligosaccharide, or a polysaccharide. The sugar may comprise one or more selected from sucrose, trehalose, galactose, mannose, maltose, lactose, fructose, and glucose.

The sugar alcohol, which is obtained by reducing an aldehyde group or a ketone group of a sugar to an alcohol group, is a generic term for a polyol having two or more hydroxy groups. The sugar alcohol may comprise one or more selected from mannitol, sorbitol, xylitol, arabitol, erythritol, lactitol, maltitol, and inositol. The sugar alcohol comprises an anhydrous form or a hydrate of the sugar alcohol. For example, trehalose may comprise not only trehalose but also trehalose dihydrate.

The concentration of the sugar may be freely adjusted within a range that maintains the stability of the antibody, and may vary individually depending on each specific sugar type. The concentration of the sugar may be any range or any value selected within about 0.1% (w/v) to about 20% (w/v). For example, the concentration of the sugar may be about 0.1% (w/v) to about 20% (w/v), about 0.1% (w/v) to about 15% (w/v), about 0.1% (w/v) to about 12% (w/v), about 0.1% (w/v) to about 10% (w/v), about 0.1% (w/v) to about 8% (w/v), about 0.1% (w/v) to about 6% (w/v), about 0.1% (w/v) to about 5.5% (w/v), about 0.1% (w/v) to about 5% (w/v), about 0.1% (w/v) to about 4.5% (w/v), about 0.1% (w/v) to about 4% (w/v), about 0.1% (w/v) to about 3.5% (w/v), about 0.1% (w/v) to about 3% (w/v), about 0.1% (w/v) to about 2.5% (w/v), about 1% (w/v) to about 20% (w/v), about 1% (w/v) to about 15% (w/v), about 1% (w/v) to about 12% (w/v), about 1% (w/v) to about 10% (w/v), about 1% (w/v) to about 8% (w/v), about 1% (w/v) to about 6% (w/v), about 1% (w/v) to about 5.5% (w/v), about 1% (w/v) to about 5% (w/v), about 1% (w/v) to about 4.5% (w/v), about 1% (w/v) to about 4% (w/v), about 1% (w/v) to about 3.5% (w/v), about 1% (w/v) to about 3% (w/v), about 1% (w/v) to about 2.5% (w/v), about 2% (w/v) to about 20% (w/v), about 2% (w/v) to about 15% (w/v), about 2% (w/v) to about 12% (w/v), about 2% (w/v) to about 10% (w/v), about 2% (w/v) to about 8% (w/v), about 2% (w/v) to about 6% (w/v), about 2% (w/v) to about 5.5% (w/v), about 2% (w/v) to about 5% (w/v), about 2% (w/v) to about 4.5% (w/v), about 2% (w/v) to about 4% (w/v), about 2% (w/v) to about 3.5% (w/v), about 2% (w/v) to about 3% (w/v), about 2% (w/v) to about 2.5% (w/v), about 3% (w/v) to about 20% (w/v), about 3% (w/v) to about 15% (w/v), about 3% (w/v) to about 12% (w/v), about 3% (w/v) to about 10% (w/v), about 3% (w/v) to about 8% (w/v), about 3% (w/v) to about 6% (w/v), about 3% (w/v) to about 5.5% (w/v), about 3% (w/v) to about 5% (w/v), about 3% (w/v) to about 4.5% (w/v), about 3% (w/v) to about 4% (w/v), about 3% (w/v) to about 3.5% (w/v), about 3.5% (w/v) to about 20% (w/v), about 3.5% (w/v) to about 15% (w/v), about 3.5% (w/v) to about 12% (w/v), about 3.5% (w/v) to about 10% (w/v), about 3.5% (w/v) to about 8% (w/v), about 3.5% (w/v) to about 6% (w/v), about 3.5% (w/v) to about 5.5% (w/v), about 3.5% (w/v) to about 5% (w/v), about 3.5% (w/v) to about 4.5% (w/v), about 3.5% (w/v) to about 4.2% (w/v), about 3.5% (w/v) to about 4% (w/v), about 3.8% (w/v) to about 12% (w/v), about 3.8% (w/v) to about 10% (w/v), about 3.8% (w/v) to about 8% (w/v), about 3.8% (w/v) to about 5% (w/v), about 3.8% (w/v) to about 4.5% (w/v), about 3.8% (w/v) to about 4.2% (w/v), about 4% (w/v) to about 20% (w/v), about 4% (w/v) to about 15% (w/v), about 4% (w/v) to about 12% (w/v), about 4% (w/v) to about 10% (w/v), about 4% (w/v) to about 8% (w/v), about 4% (w/v) to about 6% (w/v), about 4% (w/v) to about 5.5% (w/v), about 4% (w/v) to about 5% (w/v), about 4% (w/v) to about 4.5% (w/v), about 5% (w/v) to about 20% (w/v), about 5% (w/v) to about 15% (w/v), about 5% (w/v) to about 12% (w/v), about 5% (w/v) to about 10% (w/v), about 5% (w/v) to about 8% (w/v), about 5% (w/v) to about 6% (w/v), about 5% (w/v) to about 5.7% (w/v), about 5% (w/v) to about 5.5% (w/v), about 5.3% (w/v) to about 20% (w/v), about 5.3% (w/v) to about 15% (w/v), about 5.3% (w/v) to about 12% (w/v), about 5.3% (w/v) to about 10% (w/v), about 5.3% (w/v) to about 8% (w/v), or about 5.3% (w/v) to about 6% (w/v), about 5.3% (w/v) to about 5.7% (w/v), about 5.5% (w/v) to about 20% (w/v), about 5.5% (w/v) to about 15% (w/v), about 5.5% (w/v) to about 12% (w/v), about 5.5% (w/v) to about 10% (w/v), about 5.5% (w/v) to about 8% (w/v), or about 5.5% (w/v) to about 6% (w/v). The concentration of the sugar alcohol may be freely adjusted within a range that maintains the stability of the antibody, and may vary individually depending on each specific sugar alcohol type. The concentration of the sugar alcohol may be any range or any value selected within about 0.1% (w/v) to about 20% (w/v). For example, the concentration of the sugar alcohol may be about 0.1% (w/v) to about 20% (w/v), about 0.1% (w/v) to about 15% (w/v), about 0.1% (w/v) to about 12% (w/v), about 0.1% (w/v) to about 10% (w/v), about 0.1% (w/v) to about 8% (w/v), about 0.1% (w/v) to about 5% (w/v), about 0.1% (w/v) to about 4% (w/v), about 1% (w/v) to about 20% (w/v), about 1% (w/v) to about 15% (w/v), about 1% (w/v) to about 12% (w/v), about 1% (w/v) to about 10% (w/v), about 1% (w/v) to about 8% (w/v), about 1% (w/v) to about 7% (w/v), about 1% (w/v) to about 6% (w/v), about 1% (w/v) to about 5% (w/v), about 1% (w/v) to about 4% (w/v), about 1% (w/v) to about 3% (w/v), or about 1% (w/v) to about 2% (w/v).

In an embodiment, the saccharide may comprise one or more selected from sucrose, trehalose, and mannitol.

In a specific embodiment, the saccharide may comprise sucrose. In a specific embodiment, the saccharide may be sucrose.

The concentration of the sucrose may be any range or any value selected within about 0.1% (w/v) to about 20% (w/v). For example, the concentration of the sucrose may be about 0.1% (w/v) to about 20% (w/v), about 0.1% (w/v) to about 15% (w/v), about 0.1% (w/v) to about 12% (w/v), about 0.1% (w/v) to about 10% (w/v), about 0.1% (w/v) to about 8% (w/v), about 0.1% (w/v) to about 6% (w/v), about 0.1% (w/v) to about 5.5% (w/v), about 0.1% (w/v) to about 5% (w/v), about 0.1% (w/v) to about 4.5% (w/v), about 0.1% (w/v) to about 4% (w/v), about 0.1% (w/v) to about 3.5% (w/v), about 0.1% (w/v) to about 3% (w/v), about 0.1% (w/v) to about 2.5% (w/v), about 1% (w/v) to about 20% (w/v), about 1% (w/v) to about 15% (w/v), about 1% (w/v) to about 12% (w/v), about 1% (w/v) to about 10% (w/v), about 1% (w/v) to about 8% (w/v), about 1% (w/v) to about 6% (w/v), about 1% (w/v) to about 5.5% (w/v), about 1% (w/v) to about 5% (w/v), about 1% (w/v) to about 4.5% (w/v), about 1% (w/v) to about 4% (w/v), about 1% (w/v) to about 3.5% (w/v), about 1% (w/v) to about 3% (w/v), about 1% (w/v) to about 2.5% (w/v), about 2% (w/v) to about 20% (w/v), about 2% (w/v) to about 15% (w/v), about 2% (w/v) to about 12% (w/v), about 2% (w/v) to about 10% (w/v), about 2% (w/v) to about 8% (w/v), about 2% (w/v) to about 6% (w/v), about 2% (w/v) to about 5.5% (w/v), about 2% (w/v) to about 5% (w/v), about 2% (w/v) to about 4.5% (w/v), about 2% (w/v) to about 4% (w/v), about 2% (w/v) to about 3.5% (w/v), about 2% (w/v) to about 3% (w/v), about 2% (w/v) to about 2.5% (w/v), about 3% (w/v) to about 20% (w/v), about 3% (w/v) to about 15% (w/v), about 3% (w/v) to about 12% (w/v), about 3% (w/v) to about 10% (w/v), about 3% (w/v) to about 8% (w/v), about 3% (w/v) to about 6% (w/v), about 3% (w/v) to about 5.5% (w/v), about 3% (w/v) to about 5% (w/v), about 3% (w/v) to about 4.5% (w/v), about 3% (w/v) to about 4% (w/v), about 3% (w/v) to about 3.5% (w/v), about 3.5% (w/v) to about 20% (w/v), about 3.5% (w/v) to about 15% (w/v), about 3.5% (w/v) to about 12% (w/v), about 3.5% (w/v) to about 10% (w/v), about 3.5% (w/v) to about 8% (w/v), about 3.5% (w/v) to about 6% (w/v), about 3.5% (w/v) to about 5.5% (w/v), about 3.5% (w/v) to about 5% (w/v), about 3.5% (w/v) to about 4.5% (w/v), about 3.5% (w/v) to about 4.2% (w/v), about 3.5% (w/v) to about 4% (w/v), about 3.8% (w/v) to about 12% (w/v), about 3.8% (w/v) to about 10% (w/v), about 3.8% (w/v) to about 8% (w/v), about 3.8% (w/v) to about 5% (w/v), about 3.8% (w/v) to about 4.5% (w/v), about 3.8% (w/v) to about 4.2% (w/v), about 4% (w/v) to about 20% (w/v), about 4% (w/v) to about 15% (w/v), about 4% (w/v) to about 12% (w/v), about 4% (w/v) to about 10% (w/v), about 4% (w/v) to about 8% (w/v), about 4% (w/v) to about 6% (w/v), about 4% (w/v) to about 5.5% (w/v), about 4% (w/v) to about 5% (w/v), about 4% (w/v) to about 4.5% (w/v), about 5% (w/v) to about 20% (w/v), about 5% (w/v) to about 15% (w/v), about 5% (w/v) to about 12% (w/v), about 5% (w/v) to about 10% (w/v), about 5% (w/v) to about 8% (w/v), about 5% (w/v) to about 6% (w/v), about 5% (w/v) to about 5.7% (w/v), about 5% (w/v) to about 5.5% (w/v), about 5.3% (w/v) to about 20% (w/v), about 5.3% (w/v) to about 15% (w/v), about 5.3% (w/v) to about 12% (w/v), about 5.3% (w/v) to about 10% (w/v), about 5.3% (w/v) to about 8% (w/v), or about 5.3% (w/v) to about 6% (w/v), about 5.3% (w/v) to about 5.7% (w/v), about 5.5% (w/v) to about 20% (w/v), about 5.5% (w/v) to about 15% (w/v), about 5.5% (w/v) to about 12% (w/v), about 5.5% (w/v) to about 10% (w/v), about 5.5% (w/v) to about 8% (w/v), or about 5.5% (w/v) to about 6% (w/v).In an embodiment, the concentration of the sucrose may be about 2% (w/v) to about 6% (w/v).

In a specific embodiment, the concentration of the sucrose may be about 4% (w/v).

In a specific embodiment, the concentration of the sucrose may be about 5.5% (w/v).

### (4) Amino Acid

The liquid formulation according to one aspect comprises an amino acid.

The liquid formulation according to one aspect comprises methionine.

The concentration of the methionine may be about 1 mM to about 400 mM, about 2.7 mM to about 400 mM, about 2.7 mM to about 300 mM, about 2.7 mM to about 200 mM, about 2.7 mM to about 100 mM, about 2.7 mM to about 90 mM, about 2.7 mM to about 50 mM, about 2.7 mM to about 40 mM, about 2.7 mM to about 35 mM, about 2.7 mM to about 30 mM, about 10 mM to about 400 mM, about 10 mM to about 300 mM, about 10 mM to about 200 mM, about 10 mM to about 100 mM, about 10 mM to about 90 mM, about 10 mM to about 50 mM, about 10 mM to about 40 mM, about 10 mM to about 35 mM, about 10 mM to about 30 mM, about 15 mM to about 100 mM, about 15 mM to about 50 mM, about 15 mM to about 40 mM, about 15 mM to about 35 mM, about 15 mM to about 30 mM, about 20 mM to about 100 mM, about 20 mM to about 90 mM, about 20 mM to about 80 mM, about 20 mM to about 70 mM, about 20 mM to about 60 mM, about 20 mM to about 50 mM, about 20 mM to about 40 mM, about 20 mM to about 35 mM, about 20 mM to about 30 mM, about 20 mM to about 28 mM, about 22 mM to about 100 mM, about 22 mM to about 90 mM, about 22 mM to about 50 mM, about 22 mM to about 40 mM, about 22 mM to about 35 mM, about 22 mM to about 30 mM, about 22 mM to about 28 mM, about 25 mM to about 100 mM, about 25 mM to about 90 mM, about 25 mM to about 50 mM, about 25 mM to about 40 mM, about 25 mM to about 35 mM, or about 25 mM to about 30 mM.

In an embodiment, the concentration of the methionine may be any range or any value selected within about 2.7 mM to about 90 mM.

In an embodiment, the concentration of the methionine may be about 20 mM to about 90 mM.

In a specific embodiment, the concentration of the methionine may be about 20 mM to about 30 mM.

In a specific embodiment, the concentration of the methionine may be about 25 mM.

The methionine may have one or more functions of a viscosity reducing agent, a stabilizer, and an antioxidant. The methionine may have all the functions of a viscosity reducing agent, a stabilizer, and an antioxidant.

The term "viscosity reducing agent" or "viscosity reducer" refers to a substance that lowers the viscosity of a liquid substance.

The term "stabilizer" refers to a substance added to prevent a state change or a chemical change when preserving a substance. The stabilizer may be a thermal stabilizer. The stabilizer may inhibit aggregation or denaturation of the antibody.

The term "antioxidant" is a generic term for a substance that prevents oxidation, and specifically, as used herein, may refer to a substance that prevents oxidation of a protein (e.g., an antibody).

The methionine may increase the aggregation temperature (T_{agg}) of the antibody in the liquid formulation. When the anti-IL-4Rα antibody-containing liquid formulation includes methionine, the liquid formulation may exhibit a higher T_{agg} compared to a case where it includes arginine or histidine. Therefore, the methionine may increase the thermal stability of the anti-IL-4Rα antibody-containing liquid formulation.

The methionine may increase the diffusion interaction parameter (K_{D}) of the liquid formulation. When the anti-IL-4Rα antibody-containing liquid formulation includes methionine, said formulation may exhibit a higher K_{D} compared to a formulation containing sucrose, trehalose, sorbitol, mannitol, arginine, lysine, histidine, or NaCl. Therefore, the methionine may increase the chemical stability of the anti-IL-4Rα antibody-containing liquid formulation by inhibiting aggregation of the anti-IL-4Rα antibody in the liquid formulation.

The methionine may decrease the viscosity (cP) of the liquid formulation. When the anti-IL-4Rα antibody-containing liquid formulation includes methionine, the liquid formulation may have a lower viscosity compared to a case where the formulation is free of methionine. When methionine is added to the anti-IL-4Rα antibody-containing liquid formulation containing saccharide, the liquid formulation may have a lower viscosity compared to a case where methionine is not added. When lysine is replaced with methionine in the anti-IL-4Rα antibody-containing liquid formulation containing saccharide and lysine, the liquid formulation may have a lower viscosity compared to a case where lysine is not replaced with methionine. Therefore, because the methionine decreases the viscosity of the anti-IL-4Rα antibody-containing liquid formulation, the methionine may be used as a viscosity reducing agent.

The methionine may decrease the high molecular weight species content (%HMW) of the antibody in the liquid formulation. When the anti-IL-4Rα antibody-containing liquid formulation includes methionine, the %HMW of the antibody in the liquid formulation may be decreased upon storage under the same conditions (e.g., 4 weeks under accelerated stability conditions of 25±2 °C and 60±5% RH, or 4 weeks under stress stability conditions of 40±2 °C and 75+5% RH) compared to a case where the formulation is free of methionine. When methionine is added to the anti-IL-4Rα antibody-containing liquid formulation including a saccharide, the %HMW of the antibody in the liquid formulation may be decreased upon storage under the same conditions (e.g., 4 weeks under accelerated stability conditions of 25±2 °C and 60±5% RH, or 4 weeks under stress stability conditions of 40±2 °C and 75+5% RH) compared to a case where methionine is not added. When lysine is replaced with methionine in the anti-IL-4Rα antibody-containing liquid formulation containing saccharide and lysine, the %HMW of the antibody in the liquid formulation may be decreased upon storage under the same conditions (e.g., 4 weeks under accelerated stability conditions of 25±2 °C and 60±5% RH, or 4 weeks under stress stability conditions of 40±2 °C and 75+5% RH) compared to a case where lysine is not replaced with methionine. Therefore, the methionine may increase the stability of the anti-IL-4Rα antibody-containing liquid formulation by inhibiting aggregation of the anti-IL-4Rα antibody in the liquid formulation.

The methionine may decrease the acidic species content (%Acidic) of the antibody in the liquid formulation. When the anti-IL-4Rα antibody-containing liquid formulation includes methionine, the %Acidic of the antibody in the liquid formulation may be decreased upon storage under the same conditions (e.g., 4 weeks under accelerated stability conditions of 25±2 °C and 60±5% RH) compared to a case where the formulation is free of methionine. Therefore, the methionine may increase the stability of the anti-IL-4Rα antibody-containing liquid formulation by decreasing generation of acidic species of the anti-IL-4Rα antibody in the liquid formulation.

The methionine may decrease the oxidation level (%Met₂₅₇) of the methionine residue in the antibody in the liquid formulation. When the anti-IL-4Rα antibody-containing liquid formulation includes methionine, the %Met₂₅₇ of the antibody in the liquid formulation may be decreased upon storage under the same conditions (e.g., 4 weeks under accelerated stability conditions of 25±2 °C and 60±5% RH) compared to a case where the formulation is free of methionine. Therefore, the methionine may increase the stability of the anti-IL-4Rα antibody-containing liquid formulation by inhibiting oxidation of the anti-IL-4Rα antibody in the liquid formulation.

The liquid formulation according to one aspect may further comprise histidine. Therefore, the liquid formulation may be a liquid formulation comprising an anti-IL-4Rα antibody, a buffer, a saccharide, methionine, and histidine, and having a pH of about 4.9 to about 5.5.

The concentration of the histidine may be about 0.1 mM to about 400 mM, about 0.1 mM to about 92.4 mM, about 0.5 mM to about 400 mM, about 0.5 mM to about 92.4 mM, about 1 mM to about 400 mM, about 10 mM to about 400 mM, about 20 mM to about 400 mM, about 20 mM to about 300 mM, about 20 mM to about 200 mM, about 20 mM to about 100 mM, or about 20 mM to about 92.4 mM.

In an embodiment, the concentration of the histidine may be any range or any value selected within about 20 mM to about 92.4 mM. For example, the concentration of the histidine may be about 20 mM to about 92.4 mM, about 20 mM to about 90 mM, about 20 mM to about 80 mM, about 20 mM to about 60 mM, about 30 mM to about 92.4 mM, about 30 mM to about 90 mM, about 30 mM to about 80 mM, about 30 mM to about 60 mM, about 40 mM to about 92.4 mM, about 40 mM to about 90 mM, about 40 mM to about 80 mM, about 40 mM to about 60 mM, about 45 mM to about 92.4 mM, about 45 mM to about 90 mM, about 45 mM to about 80 mM, about 45 mM to about 60 mM, about 45 mM to about 55 mM, about 50 mM to about 92.4 mM, about 50 mM to about 90 mM, about 50 mM to about 80 mM, or about 50 mM to about 60 mM.

In an embodiment, the histidine may have a function of a buffer.

In an embodiment, the histidine may have a function of a stabilizer.

The histidine may decrease the acidic species content (%Acidic) of the antibody in the liquid formulation. When the anti-IL-4Rα antibody-containing liquid formulation includes histidine, the %Acidic of the antibody in the liquid formulation may be decreased upon storage under the same conditions (e.g., 4 weeks under accelerated stability conditions of 25±2 °C and 60±5% RH) compared to a case where the formulation is free of histidine. Therefore, the histidine may increase the stability of the anti-IL-4Rα antibody-containing liquid formulation by decreasing generation of acidic species of the anti-IL-4Rα antibody in the liquid formulation.

The histidine may decrease the oxidation level (%Met₂₅₇) of the methionine residue in the antibody in the liquid formulation. When the anti-IL-4Rα antibody-containing liquid formulation includes histidine, the %Met₂₅₇ of the antibody in the liquid formulation may be decreased upon storage under the same conditions (e.g., 4 weeks under accelerated stability conditions of 25±2 °C and 60±5% RH) compared to a case where the formulation is free of histidine. Therefore, the histidine may increase the stability of the anti-IL-4Rα antibody-containing liquid formulation by inhibiting oxidation of the anti-IL-4Rα antibody in the liquid formulation.

The liquid formulation according to one aspect may be free of arginine. The liquid formulation may not comprise arginine as a viscosity reducing agent. The arginine may comprise arginine or a salt thereof, for example, arginine hydrochloride.

The liquid formulation according to one aspect may be free of amino acids other than methionine and histidine.

The term "free of component A" or "substantially free of component A" may be interpreted to include a case where component A is not present at all, a case where, even when component A is present, it is present in a trace amount that does not substantially affect the properties of the formulation, or a case where it is present in an undetectable amount.

As used herein, the phrase "free of arginine" may be interpreted to mean that an arginine component is not present in the formulation, or that arginine is included in an amount insufficient to perform its intended function as a viscosity reducing agent in the formulation.

Because the liquid formulation includes methionine instead of arginine, both viscosity and %HMW are decreased compared to Dupixent containing arginine, and thus viscosity and stability may be simultaneously improved.

### (5) Surfactant

The liquid formulation according to one aspect may further comprise a surfactant.

Therefore, the liquid formulation may be a liquid formulation comprising an anti-IL-4Rα antibody, a buffer, a saccharide, methionine, and a surfactant, and having a pH of about 4.9 to about 5.5.

The liquid formulation may be a liquid formulation comprising an anti-IL-4Rα antibody, a buffer, a saccharide, methionine, histidine, and a surfactant, and having a pH of about 4.9 to about 5.5.

The surfactant may be selected from any pharmaceutically acceptable surfactants capable of evenly dispersing a protein (e.g., an antibody) in a liquid formulation medium.

The surfactant may be a nonionic surfactant.

Specifically, the surfactant may be one or more selected from the group consisting of a polysorbate, a poloxamer, a sorbitan ester of another fatty acid, a polyethylene-polypropylene glycol, a polyoxyethylene compound, sodium dodecyl sulphate (SDS), and the like.

The polysorbate may comprise polysorbate 20, polysorbate 40, polysorbate 60, polysorbate 65, polysorbate 80, and polysorbate 85, and the like.

The poloxamer may comprise a PEO-PPO-PEO copolymer (PEO is poly(ethylene oxide) and PPO is poly(propylene oxide)), and the like.

The sorbitan ester of another fatty acid may refer to a sorbitan ester of a fatty acid other than a polysorbate, and may comprise, for example, a sorbitan polyethoxylate, and the like.

The polyoxyethylene compound may comprise polyoxyethylene stearate, a polyoxyethylene alkyl ether (alkyl: C1-C30), polyoxyethylene monolauryl ether, an alkylphenyl polyoxyethylene copolymer (alkyl: C1-C30), and the like.

In an embodiment, the surfactant may be a polysorbate.

In an embodiment, the surfactant may comprise one or more selected from polysorbate 20, polysorbate 40, polysorbate 60, polysorbate 65, polysorbate 80, and polysorbate 85.

In an embodiment, the surfactant may comprise polysorbate 20, polysorbate 80, or a combination thereof.

In a specific embodiment, the surfactant may comprise polysorbate 80. In a specific embodiment, the surfactant may be polysorbate 80.

The concentration of the surfactant may be any range or any value selected within about 0.01% (w/v) to about 0.9% (w/v). For example, the concentration of the surfactant may be about 0.01% (w/v) to about 0.9% (w/v), about 0.01% (w/v) to about 0.5% (w/v), about 0.1% (w/v) to about 0.9% (w/v), about 0.1% (w/v) to about 0.5% (w/v), about 0.1% (w/v) to about 0.4% (w/v), about 0.1% (w/v) to about 0.3% (w/v), about 0.15% (w/v) to about 0.9% (w/v), about 0.15% (w/v) to about 0.5% (w/v), about 0.15% (w/v) to about 0.4% (w/v), about 0.15% (w/v) to about 0.3% (w/v), or about 0.15% (w/v) to about 0.25% (w/v).

In a specific embodiment, the concentration of the surfactant may be about 0.2% (w/v).

### (6) Diluent

The liquid formulation according to one aspect may further comprise a diluent.

The diluent may be an aqueous carrier. The aqueous carrier is a pharmaceutically acceptable carrier that is safe and non-toxic when it is administered to a human, and may be, for example, water, a saline solution, a Ringer's solution, dextrose, or a mixture thereof.

In an embodiment, the diluent may be water. Therefore, the liquid formulation may be an aqueous liquid formulation.

### (7) Formulation

The term "liquid formulation" refers to a formulation in a liquid state.

The liquid formulation according to one aspect is a stable liquid formulation of an anti-IL-4Rα antibody.

The liquid formulation according to one aspect may be a pharmaceutical formulation of an anti-IL-4Rα antibody.

The term "pharmaceutical composition" or "pharmaceutical preparation" refers to a preparation that allows the biological activity of an active ingredient to act effectively, and does not include an additional ingredient having severe toxicity to a subject to which the preparation is to be administered.

The term "pharmaceutical formulation" refers to a product of a process of producing a final drug product by combining an active drug with a chemical substance.

The term "pharmaceutically acceptable" may refer to excipients, carriers, vehicles, diluents, additives, salts, and the like suitable for administration to a subject.

The liquid formulation according to one aspect may be a Dupixent^{®} biosimilar. The liquid formulation according to one aspect has improved stability and improved viscosity compared to Dupixent.

The term "biosimilar", also called a "biogeneric", refers to a generic drug for an original biopharmaceutical. Because a biopharmaceutical is not synthesized as a chemical product but is produced through cells, a product perfectly identical to the original drug cannot be replicated. Therefore, a generic drug for a biopharmaceutical is called a biosimilar in the sense that it is similar to, although not identical to, the original drug.

The liquid formulation according to one aspect may be selected from the following items:
1) A liquid formulation comprising an anti-IL-4Rα antibody, a buffer, a saccharide, and methionine, and having a pH of about 4.9 to about 5.5;
2) A liquid formulation comprising an anti-IL-4Rα antibody, a buffer, a saccharide, methionine, and histidine, and having a pH of about 4.9 to about 5.5;
3) A liquid formulation comprising an anti-IL-4Rα antibody, a buffer, a saccharide, methionine, and a surfactant, and having a pH of about 4.9 to about 5.5;
4) A liquid formulation comprising an anti-IL-4Rα antibody, a buffer, a saccharide, methionine, histidine, and a surfactant, and having a pH of about 4.9 to about 5.5;
5) A liquid formulation comprising about 5 mg/mL to about 300 mg/mL of dupilumab, about 10 mM to about 40 mM of acetate, about 2% (w/v) to about 10% (w/v) of sucrose, about 10 mM to about 100 mM of methionine, about 20 mM to about 92.4 mM of histidine, and about 0.1% (w/v) to about 0.9% (w/v) of polysorbate 80, and having a pH of about 4.9 to about 5.5;
6) A liquid formulation comprising 100 mg/mL to 200 mg/mL of dupilumab, 30 mM±5 mM of acetate, 4%±1.0% (w/v) of sucrose, 25 mM±5 mM of methionine, 20 mM to 92.4 mM of histidine, and 0.2%±0.1% (w/v) of polysorbate 80, and having a pH of about 4.9 to about 5.5;
7) A liquid formulation comprising 175 mg/mL of dupilumab, 30 mM±5 mM of acetate, 4%±1.0% (w/v) of sucrose, 25 mM±5 mM of methionine, 20 mM to 92.4 mM of histidine, and 0.2%±0.1% (w/v) of polysorbate 80, and having a pH of about 4.9 to about 5.5;
8) A liquid formulation comprising 175 mg/mL of dupilumab, 30 mM±5 mM of acetate, 4%±1.0% (w/v) of sucrose, 25 mM±5 mM of methionine, 20 mM±5 mM of histidine, and 0.2%±0.1% (w/v) of polysorbate 80, and having a pH of about 4.9;
9) A liquid formulation comprising 175 mg/mL of dupilumab, 30 mM±5 mM of acetate, 4%±1.0% (w/v) of sucrose, 25 mM±5 mM of methionine, 50 mM±5 mM of histidine, and 0.2%±0.1% (w/v) of polysorbate 80, and having a pH of about 4.8;
10) A liquid formulation comprising 175 mg/mL of dupilumab, 30 mM±5 mM of acetate, 4%±1.0% (w/v) of sucrose, 25 mM±5 mM of methionine, 50 mM±5 mM of histidine, and 0.2%±0.1% (w/v) of polysorbate 80, and having a pH of about 5.2;
11) A liquid formulation comprising 175 mg/mL of dupilumab, 30 mM±5 mM of acetate, 4%±1.0% (w/v) of sucrose, 25 mM±5 mM of methionine, 80 mM±5 mM of histidine, and 0.2%±0.1% (w/v) of polysorbate 80, and having a pH of about 4.9;
12) A liquid formulation comprising 175 mg/mL of dupilumab, 30 mM±5 mM of acetate, 4%±1.0% (w/v) of sucrose, 25 mM±5 mM of methionine, 80 mM±5 mM of histidine, and 0.2%±0.1% (w/v) of polysorbate 80, and having a pH of about 5.5;
13) A liquid formulation comprising 175 mg/mL of dupilumab, 30 mM±5 mM of acetate, 4%±1.0% (w/v) of sucrose, 25 mM±5 mM of methionine, 92.4 mM±5 mM of histidine, and 0.2%±0.1% (w/v) of polysorbate 80, and having a pH of about 5.2;
14) A liquid formulation comprising 100 mg/mL to 200 mg/mL of dupilumab, 30 mM±5 mM of acetate, 5.5%±1.0% (w/v) of sucrose, 25 mM±5 mM of methionine, 20 mM to 92.4 mM of histidine, and 0.2%±0.1% (w/v) of polysorbate 80, and having a pH of about 4.9 to about 5.5;
15) A liquid formulation comprising 175 mg/mL of dupilumab, 30 mM±5 mM of acetate, 5.5%±1.0% (w/v) of sucrose, 25 mM±5 mM of methionine, 20 mM to 92.4 mM of histidine, and 0.2%±0.1% (w/v) of polysorbate 80, and having a pH of about 4.9 to about 5.5;
16) A liquid formulation comprising 175 mg/mL of dupilumab, 30 mM±5 mM of acetate, 5.5%±1.0% (w/v) of sucrose, 25 mM±5 mM of methionine, 20 mM±5 mM of histidine, and 0.2%±0.1% (w/v) of polysorbate 80, and having a pH of about 4.9;
17) A liquid formulation comprising 175 mg/mL of dupilumab, 30 mM±5 mM of acetate, 5.5%±1.0% (w/v) of sucrose, 25 mM±5 mM of methionine, 50 mM±5 mM of histidine, and 0.2%±0.1% (w/v) of polysorbate 80, and having a pH of about 4.8;
18) A liquid formulation comprising 175 mg/mL of dupilumab, 30 mM±5 mM of acetate, 5.5%±1.0% (w/v) of sucrose, 25 mM±5 mM of methionine, 50 mM±5 mM of histidine, and 0.2%±0.1% (w/v) of polysorbate 80, and having a pH of about 5.2;
19) A liquid formulation comprising 175 mg/mL of dupilumab, 30 mM±5 mM of acetate, 5.5%±1.0% (w/v) of sucrose, 25 mM±5 mM of methionine, 80 mM±5 mM of histidine, and 0.2%±0.1% (w/v) of polysorbate 80, and having a pH of about 4.9;
20) A liquid formulation comprising 175 mg/mL of dupilumab, 30 mM±5 mM of acetate, 5.5%±1.0% (w/v) of sucrose, 25 mM±5 mM of methionine, 80 mM±5 mM of histidine, and 0.2%±0.1% (w/v) of polysorbate 80, and having a pH of about 5.5; or
21) A liquid formulation comprising 175 mg/mL of dupilumab, 30 mM±5 mM of acetate, 5.5%±1.0% (w/v) of sucrose, 25 mM±5 mM of methionine, 92.4 mM±5 mM of histidine, and 0.2%±0.1% (w/v) of polysorbate 80, and having a pH of about 5.2.

### (8) Stability and Viscosity

The liquid formulation according to one aspect may secure the stability of the antibody while simultaneously having a low viscosity.

In the Examples of the present disclosure, three experiments were performed. First, the superiority of methionine among excipients was confirmed by comparatively evaluating thermal stability and chemical stability depending on the type of excipient through T_{agg} and K_{D} measurements. Second, the functions of methionine as a viscosity reducing agent, a stabilizer, and an antioxidant were confirmed by evaluating viscosity, %HMW, %Acidic, and %Met₂₅₇. Third, through a comparative evaluation of viscosity and %HMW with the Dupixent formulation, it was confirmed that the methionine-containing anti-IL-4Rα antibody liquid formulation can simultaneously improve stability and viscosity compared to a commercialized formulation.

The term "stability" means that the antibody (e.g., dupilumab) included in the formulation substantially retains its physical stability, chemical stability, and/or biological activity before and after administration, during an additional manufacturing process, or upon preservation or storage. For example, it may be understood to mean that the degree of loss of stability, such as aggregation, degradation, denaturation (acidic or basic), oxidation, and the like of the antibody included in the formulation, is 20 % or less, 15 % or less, 10 % or less, or 5 % or less compared to the initial stage of storage. Therefore, "excellent stability" or "improved stability" may mean a low protein aggregation rate, a low protein degradation rate, a low protein denaturation rate, a low amino acid oxidation rate, and the like during storage. Physical stability, chemical stability, and/or biological activity may be evaluated by commonly known methods.

As a method for evaluating the stability of the antibody included in the formulation in a short period of time, there is a method of measuring an aggregation temperature (T_{agg}), a diffusion interaction parameter (K_{D}), and the like after exposing the formulation to a factor causing structural and physicochemical denaturation of a protein, such as heat (temperature) and the like. For example, "improved stability" may mean that the T_{agg} or K_{D} value measured for the formulation is increased by 0.01 % or more, 0.1 % or more, 0.5 % or more, 1 % or more, 2 % or more, 3 % or more, 4 % or more, 5 % or more, 10 % or more, 15 % or more, or 20 % or more compared to an existing formulation. An increase in the T_{agg} or K_{D} value may indicate improved stability, as it reflects a reduced propensity for antibody aggregation in the formulation due to an external factor (e.g., heat, temperature).

The term "aggregate" may refer to a high molecular weight (HMW) species formed by aggregation of an antibody protein. The term "protein aggregation rate" may be expressed as a high molecular weight species content (%HMW) of the antibody in the formulation at a certain time point. The %HMW may be measured by size exclusion chromatography (SEC), but is not limited thereto. For example, "improved stability" may mean that the %HMW of the antibody measured for the formulation is decreased by 0.01 % or more, 0.1 % or more, 0.5 % or more, 1 % or more, 2 % or more, 3 % or more, 4 % or more, 5 % or more, 10 % or more, 15 % or more, or 20 % or more compared to an existing formulation. A decrease in the %HMW may mean that the stability is improved because the degree to which aggregation of the antibody in the formulation occurs is decreased.

The term "protein denaturation rate" may be expressed as an acidic species content (%Acidic) of the antibody in the formulation at a certain time point. The %Acidic may be measured by imaged capillary isoelectric focusing (icIEF), but is not limited thereto. For example, "improved stability" may mean that the %Acidic of the antibody measured for the formulation is decreased by 0.01 % or more, 0.1 % or more, 0.5 % or more, 1 % or more, 2 % or more, 3 % or more, 4 % or more, 5 % or more, 10 % or more, 15 % or more, or 20 % or more compared to an existing formulation. A decrease in the %Acidic may mean that the stability is improved because the degree to which denaturation of the antibody in the formulation occurs is decreased.

The term "amino acid oxidation rate" may refer to a ratio of oxidized amino acids (e.g., a ratio of oxidized methionine) among the amino acids of the antibody protein. In an embodiment, the amino acid oxidation rate may refer to an oxidation rate (%Met₂₅₇) of methionine (Met₂₅₇), which is the 257th amino acid residue of dupilumab, an anti-IL-4Rα antibody. The %Met₂₅₇ may be measured by liquid chromatography-mass spectrometry (LC-MS), but is not limited thereto. For example, "improved stability" may mean that the %Met₂₅₇ of dupilumab measured for the formulation is decreased by 0.01 % or more, 0.1 % or more, 0.5 % or more, 1 % or more, 2 % or more, 3 % or more, 4 % or more, 5 % or more, 10 % or more, 15 % or more, or 20 % or more compared to an existing formulation. A decrease in the %Met₂₅₇ may mean that the stability is improved because the degree to which oxidation of the antibody in the formulation occurs is decreased.

The stability evaluation may be performed immediately after preparation of the formulation, or after storage for a certain period of time under accelerated stability conditions or stress stability conditions. The accelerated stability conditions may include conditions used in an accelerated test for pharmaceuticals, and may be, for example, a temperature of 25±2 °C and a relative humidity (RH) of 60±5 %. The stress stability conditions may include conditions used in a stress test for pharmaceuticals, and may be, for example, a temperature of 40±2 °C and a relative humidity (RH) of 75+5 %.

The liquid formulation may have an increased aggregation temperature (T_{agg}).

By including methionine, the liquid formulation may have an increased T_{agg} compared to an anti-IL-4Rα antibody liquid formulation including arginine.

The liquid formulation may have an increased diffusion interaction parameter (K_{D}).

By including methionine, the liquid formulation may have an increased K_{D} compared to an anti-IL-4Rα antibody liquid formulation including arginine. By including methionine, the liquid formulation may have an increased K_{D} compared to an anti-IL-4Rα antibody liquid formulation including sucrose, trehalose, sorbitol, mannitol, arginine, lysine, histidine, or NaCl. By including methionine, the liquid formulation may have an increased K_{D} compared to an anti-IL-4Rα antibody liquid formulation including a dual buffer of 12.5 mM acetate and 20 mM histidine, a pH of 5.9, 5% (w/v) sucrose, and 50 mM arginine.

The liquid formulation may have an improved viscosity. The 'improvement' of the viscosity may mean a 'decrease' in the viscosity. Therefore, the liquid formulation may have a decreased viscosity.

By including methionine, the liquid formulation may have an improved viscosity compared to an anti-IL-4Rα antibody liquid formulation not including methionine.

In an Example, it was confirmed that the viscosity is improved when methionine is added to a saccharide-containing anti-IL-4Rα antibody liquid formulation. In addition, in a saccharide-containing anti-IL-4Rα antibody liquid formulation, it was confirmed that methionine has a superior function as a viscosity reducing agent compared to lysine.

The liquid formulation according to one aspect may have a decreased viscosity compared to Dupixent. In an embodiment, the liquid formulation according to one aspect may have a decreased viscosity compared to a viscosity of 11.1 cP (centipoise) of Dupixent 200 mg. In another embodiment, the liquid formulation according to one aspect may have a decreased viscosity compared to a viscosity of Dupixent 300 mg.

The viscosity of the liquid formulation may be less than 11.1 cP (centipoise). The viscosity of the liquid formulation may be less than 11.1 cP, 11.0 cP or less, 10.5 cP or less, 10.0 cP or less, 9.5 cP or less, 9.0 cP or less, 8.5 cP or less, 8.0 cP or less, 7.5 cP or less, 7.0 cP or less, or 6.5 cP or less. The viscosity of the liquid formulation may be 5.0 cP or more to less than 11.1 cP, 5.0 cP to 11.0 cP, 5.0 cP to 10.5 cP, 5.0 cP to 10.0 cP, 5.0 cP to 9.5 cP, 5.0 cP to 9.0 cP, 5.0 cP to 8.5 cP, 5.0 cP to 8.0 cP, 5.0 cP to 7.5 cP, 5.0 cP to 7.0 cP, 5.0 cP to 6.5 cP, 5.5 cP or more to 11.1 cP or less, 5.5 cP to 11.0 cP, 5.5 cP to 10.5 cP, 5.5 cP to 10.0 cP, 5.5 cP to 9.5 cP, 5.5 cP to 9.0 cP, 5.5 cP to 8.5 cP, 5.5 cP to 8.0 cP, 5.5 cP to 7.5 cP, 5.5 cP to 7.0 cP, 5.5 cP to 6.5 cP, 6.0 cP or more to 11.1 cP or less, 6.0 cP to 11.0 cP, 6.0 cP to 10.5 cP, 6.0 cP to 10.0 cP, 6.0 cP to 9.5 cP, 6.0 cP to 9.0 cP, 6.0 cP to 8.5 cP, 6.0 cP to 8.0 cP, 6.0 cP to 7.5 cP, 6.0 cP to 7.0 cP, 6.0 cP to 6.5 cP, 6.5 cP or more to 11.1 cP or less, 6.5 cP to 11.0 cP, 6.5 cP to 10.5 cP, 6.5 cP to 10.0 cP, 6.5 cP to 9.5 cP, 6.5 cP to 9.0 cP, 6.5 cP to 8.5 cP, 6.5 cP to 8.0 cP, 6.5 cP to 7.5 cP, 6.5 cP to 7.0 cP, 7.0 cP or more to 11.1 cP or less, 7.0 cP to 11.0 cP, 7.0 cP to 10.5 cP, 7.0 cP to 10.0 cP, 7.0 cP to 9.5 cP, 7.0 cP to 9.0 cP, 7.0 cP to 8.5 cP, 7.0 cP to 8.0 cP, 7.5 cP or more to 11.1 cP or less, 7.5 cP to 11.0 cP, 7.5 cP to 10.5 cP, 7.5 cP to 10.0 cP, 7.5 cP to 9.5 cP, 7.5 cP to 9.0 cP, 7.5 cP to 8.5 cP, 7.5 cP to 8.0 cP, 8.0 cP or more to 11.1 cP or less, 8.0 cP to 11.0 cP, 8.0 cP to 10.5 cP, 8.0 cP to 10.0 cP, 8.0 cP to 9.5 cP, 8.0 cP to 9.0 cP, or 8.0 cP to 8.5 cP.

In an embodiment, the viscosity of the liquid formulation may be about 6.0 cP, about 6.1 cP, about 6.2 cP, about 6.3 cP, about 6.4 cP, about 6.5 cP, about 6.6 cP, about 6.7 cP, about 6.8 cP, about 6.9 cP, about 7.0 cP, about 7.1 cP, about 7.2 cP, about 7.3 cP, about 7.4 cP, about 7.5 cP, about 7.6 cP, about 7.7 cP, about 7.8 cP, about 7.9 cP, about 8.0 cP, about 8.1 cP, about 8.2 cP, about 8.3 cP, about 8.4 cP, about 8.5 cP, about 8.6 cP, about 8.7 cP, about 8.8 cP, about 8.9 cP, about 9.0 cP, about 9.1 cP, about 9.2 cP, about 9.3 cP, about 9.4 cP, or about 9.5 cP.

The liquid formulation may have a viscosity decreased by 10 % or more, 15 % or more, 20 % or more, 25 % or more, or 30 % or more compared to the viscosity of the Dupixent formulation.

The liquid formulation may have a decreased high molecular weight species content (%HMW).

By including methionine, the liquid formulation may have a decreased %HMW compared to an anti-IL-4Rα antibody liquid formulation not including methionine.

In the liquid formulation, the %HMW of the antibody measured after storage at 40 °C for 4 weeks may decrease by 5 % or more, 10 % or more, 15 % or more, or 20 % or more compared to a formulation not including methionine.

In the liquid formulation, the %HMW of the antibody measured after storage at 25 °C for 4 weeks may decrease by 5% or more, 10% or more, or 15% or more compared to a formulation not including methionine.

In the liquid formulation, the %HMW of the antibody measured after storage at 25 °C for 4 weeks may be 3.1 % or less, 3.0 % or less, 2.9 % or less, 2.8 % or less, 2.7 % or less, 2.6 % or less, 2.5 % or less, 2.4 % or less, or 2.3 % or less.

In the liquid formulation, the %HMW of the antibody measured immediately after preparation may be less than 2.03 %, 2.02 % or less, 2.01 % or less, 2.00 % or less, 1.99 % or less, 1.98 % or less, 1.97 % or less, 1.96 % or less, 1.95 % or less, or 1.94 % or less.

The liquid formulation may have a decreased acidic species content (%Acidic).

By including methionine, the liquid formulation may have a decreased %Acidic compared to an anti-IL-4Rα antibody liquid formulation not including methionine.

In the liquid formulation, the %Acidic of the antibody measured after storage at 25 °C for 4 weeks may decrease by 1 % or more, 2 % or more, 3 % or more, 4 % or more, 5 % or more, or 7 % or more compared to a formulation not including methionine.

In the liquid formulation, the %Acidic of the antibody measured after storage at 25 °C for 4 weeks may be less than 41 %, 40 % or less, or 39 % or less.

The liquid formulation may have a decreased oxidation rate of the methionine residue (%Met₂₅₇).

By including methionine, the liquid formulation may have a decreased %Met₂₅₇ compared to an anti-IL-4Rα antibody liquid formulation not including methionine.

In the liquid formulation, the %Met₂₅₇ of the antibody measured after storage at 25 °C for 4 weeks may decrease by 50 % or more, 55 % or more, or 60 % or more compared to a formulation not including methionine.

In the liquid formulation, the %Met₂₅₇ of the antibody measured after storage at 25 °C for 4 weeks may be less than 12 %, 10 % or less, 8 % or less, 6 % or less, or 5 % or less.

### (9) Device

Another aspect provides a device comprising the liquid formulation according to one aspect.

The device is mainly used for a purpose of parenteral administration. The parenteral administration may comprise, for example, subcutaneous, intramuscular, intravenous, intraperitoneal, intracerebrospinal, intra-articular, intrasynovial, or intrathecal administration. The device may be accompanied by instructions for administration.

The device may comprise the liquid formulation in a container selected from a syringe, a pre-filled syringe, a pre-filled pen, a microinfuser, an autoinjector, a bottle, a vial, and a tube, but is not limited thereto.

The device may be in a single-dose form or a multiple-dose form.

In an embodiment, the device may comprise the liquid formulation in a pre-filled syringe. The pre-filled syringe may be a single-dose pre-filled syringe.

In an embodiment, the device may comprise the liquid formulation in a pre-filled pen. The pre-filled pen may be a single-dose pre-filled pen.

### (10) Treatment of Disease

Another aspect provides a method of treating an IL-4Rα-related condition, the method comprising administering the liquid formulation according to one aspect to a subject in need thereof. The liquid formulation may be in a form included in a device.

Another aspect provides a use of the liquid formulation according to one aspect in the manufacture of a medicament for treating an IL-4Rα-related condition.

The method for treating an IL-4Rα-related condition may further comprise identifying a subject in need of administration of an anti-IL-4Rα antibody (e.g., dupilumab) prior to the administering step.

The subject may be a subject in need of administration of a liquid formulation containing the anti-IL-4Rα antibody (e.g., dupilumab). The subject in need of administration of the liquid formulation containing the anti-IL-4Rα antibody (e.g., dupilumab) may be a subject having a disease or disorder that may be significantly treated (e.g., elimination, mitigation, alleviation, or improvement of symptoms, and the like) by administration of the anti-IL-4Rα antibody. The subject may be selected from mammals including humans.

The liquid formulation may be administered in a pharmaceutically effective amount.

The IL-4Rα-related condition may comprise any condition, disease, or disorder that may be treated by administration of the anti-IL-4Rα antibody. The IL-4Rα-related condition that may be treated by administration of an anti-IL-4Rα antibody (e.g., dupilumab) may include all indications previously approved or all indications that may be approved in the future for the anti-IL-4Rα antibody (e.g., dupilumab).

The IL-4Rα-related condition may be an IL-4-mediated disease and/or an IL-13-mediated disease. The IL-4-mediated disease may comprise any disease that may be treated by inhibition of IL-4 signaling. The IL-13-mediated disease may comprise any disease that may be treated by inhibition of IL-13 signaling. The IL-4Rα-related condition may comprise any disease that may be treated by a dual blockade of IL-4 and IL-13.

The IL-4Rα-related condition may comprise an inflammatory disease, an allergic disease, or an autoimmune disease. The inflammatory disease may be a Type 2 inflammatory disease.

The IL-4Rα-related condition may be any one selected from atopic dermatitis, asthma, chronic rhinosinusitis (CRS), chronic rhinosinusitis with nasal polyposis (CRSwNP), allergic fungal rhinosinusitis (AFRS), eosinophilic esophagitis (EoE), prurigo nodularis (PN), bullous pemphigoid (BP), chronic spontaneous urticaria (CSU), chronic inducible urticaria (CIndU), and chronic obstructive pulmonary disease (COPD), but is not limited thereto.

The atopic dermatitis may be moderate-to-severe atopic dermatitis.

The asthma may be moderate-to-severe asthma.

### (11) Route of Administration and Dosage

The liquid formulation according to one aspect may be administered by a parenteral route. The parenteral route may comprise subcutaneous administration, intravenous administration, and the like. The parenteral administration may be by a bolus injection or a continuous infusion.

In a specific embodiment, the liquid formulation may be for subcutaneous injection.

The liquid formulation may be formulated into a formulation suitable for the route of administration. For example, the liquid formulation may be formulated as an injection, an injectable ready-to-use form, and the like, but is not limited thereto.

The liquid formulation may be formulated such that the total amount or a pharmaceutically effective amount of an anti-IL-4Rα antibody (e.g., dupilumab) is provided in a single unit dosage form, or is divided across two or more (e.g., 2, 3, 4, 5, 6, 7, 8, 9, or 10) unit dosage forms. The liquid formulation may be included in a single-dose or multiple-dose form of a device.

The liquid formulation may be administered such that the total amount of the anti-IL-4Rα antibody (e.g., dupilumab) included in one unit dosage form is administered in vivo as a single bolus (e.g., within 1 minute, within 30 seconds, within 20 seconds, or within 10 seconds); or is administered as a gradual infusion in vivo over 5 minutes or more, 10 minutes or more, 30 minutes or more, 60 minutes or more, 90 minutes or more, 120 minutes or more, 150 minutes or more, 180 minutes or more, 210 minutes or more, or 240 minutes or more, but is not limited thereto.

A subject to be administered the liquid formulation may be selected from mammals comprising primates (e.g., humans, etc.), rodents (e.g., mice, rats, guinea pigs, hamsters, rabbits, etc.), cats, dogs, pigs, cows, horses, and the like.

The pharmaceutically effective amount of the liquid formulation or the anti-IL-4Rα antibody (e.g., dupilumab) included therein may mean an amount or dose sufficient to elicit a desired pharmacological effect, such as the elimination, mitigation, alleviation, or amelioration of symptoms. The pharmaceutically effective amount may be variously determined by factors such as a formulation method, a mode of administration, an age, a body weight, a sex, a pathological condition (severity of the condition) of a patient, a diet, an administration time, an administration interval, a route of administration, an excretion rate, a response sensitivity, a prior therapy, a clinical history, and the like. The dose may be adjusted at the discretion of an attending physician. The pharmaceutically effective amount may be administered at once, or divided and administered in multiple doses of two or more times.

For example, the liquid formulation may be administered once every 2 to 4 weeks over a period of 2 weeks or more at a dose such that the anti-IL-4Rα antibody (e.g., dupilumab) is 300 mg, 250 mg, 200 mg, 150 mg, or 100 mg.

The liquid formulation may be prepared as a general bulk formulation, and components of the liquid formulation are adjusted to a higher concentration than a concentration required for administration, and may be appropriately diluted and used prior to administration.

### Advantageous Effects of Invention

The liquid formulation of an anti-IL-4Rα antibody according to one aspect may exhibit improved stability and viscosity compared to existing commercial formulations. Therefore, the liquid formulation maintains high stability and low viscosity even when containing the antibody at a high concentration, thereby enhancing patient convenience upon injection. Accordingly, the liquid formulation can be usefully utilized as a medicament for treating an IL-4Rα-related condition.

### Brief Description of Drawings

FIG. 1 is a graph showing the results of measuring an aggregation temperature (T_{agg}) for 10 formulations having different types of excipients, and a formulation having a Dupixent composition.
FIG. 2 is a graph showing the results of measuring a diffusion interaction parameter (K_{D}) for 10 formulations having different types of excipients, and a formulation having a Dupixent composition.
FIG. 3 is a graph showing the results of measuring a viscosity for a methionine-containing formulation and a methionine-free formulation.
FIG. 4 is a graph showing the results of analyzing a high molecular weight species content (%HMW) for a methionine-containing formulation and a methionine-free formulation.

### Best Mode for Carrying out the Invention

### Mode for the Invention

Hereinafter, the present disclosure will be described in more detail through the following examples. However, these examples are provided for illustrative purposes, and the scope of the present disclosure is not limited to these examples.

### [Experimental Methods]

### 1. Measurement of Aggregation Temperature (T_{agg})

The aggregation temperature was measured using dynamic light scattering (DLS). Specifically, each sample was diluted with its respective buffer and loaded into a 96-well plate. The plate was loaded into a DynaPro^{®} Plate Reader^{™} II (Wyatt Technology) instrument. The size of the aggregates in the formulation was measured via DLS while increasing the temperature of the sample from 25 °C to 80 °C at a rate of 0.25 °C/min. Stability is evaluated by measuring the temperature at which the size of the aggregates changes, and a higher aggregation temperature (T_{agg}) value indicates a more stable formulation.

### 2. Analysis of Diffusion Interaction Parameter (K_{D})

The diffusion interaction parameter (K_{D}) was analyzed to evaluate whether the intermolecular forces in the fluid phase were attractive or repulsive as the protein concentration increased upon exposure of the sample to room temperature. This parameter enables the evaluation or ranking of superior formulations based on their aggregation propensity by assessing the intermolecular binding forces that lead to irreversible aggregation. Specifically, each sample was diluted with a buffer (protein concentrations: 2, 4, 6, 8, and 10 mg/mL) and loaded into a 96-well plate. The plate was loaded into a DynaPro^{®} Plate Reader^{™} II (Wyatt Technology) instrument. After measuring the diffusion coefficient of the prepared samples as a function of protein concentration, K_{D} was calculated using the slope and y-intercept of the corresponding graph. A positive K_{D} value indicates a predominance of intermolecular repulsive forces, corresponding to a low propensity for aggregation whereas a negative value indicates that attractive forces are dominant, resulting in a strong aggregation propensity; thus, a larger positive K_{D} value indicates a more stable formulation.

### 3. Measurement of Viscosity

The viscosity of the sample was measured using a viscometer (manufacturer: RheoSense; model: VROC initium one plus). The analysis was conducted at a temperature of 25 °C during the viscosity measurement. Each sample was measured across 11 segments, and the viscosity of each sample was calculated by averaging the values having a slope fit R² of 0.9995 or greater.

### 4. Analysis of high molecular weight species content (%high molecular weight species, %HMW)

The percentage of high molecular weight species (%HMW) was determined using size exclusion chromatography (SEC) from Waters. The proteins were resolved into three distinct peaks based on molecular weight, corresponding to the HMW peak (referring to protein aggregation), the monomer peak, and the LMW peak (referring to protein degradation) in ascending order of retention time (i.e., in descending order of protein molecular weight). $- % HMW = \left\{area of HMW/area of \left(HMW+monomer+LMW\right)\right\} \times 100$

For stability testing, each formulation sample was stored in a chamber maintained under accelerated stability conditions (25±2 °C and 60±5 % relative humidity (RH)) or under stress stability conditions (40±2 °C and 75±5 % RH), respectively.

### 5. Analysis of acidic species content (%Acidic)

The %Acidic value of the sample was measured using imaging capillary isoelectric focusing (icIEF). The samples were injected into an iCE3 system (ProteinSimple, USA), and the isoelectric point (pI) and the relative area of the protein charge variants were measured using iCE CFR software. The %Acidic represents the relative area of the protein charge variants detected prior to the main peak. Quantifying these parameters allows for the identification of the protein charge variants. When a protein undergoes stress-induced denaturation, it may form aggregates or exhibit an altered charge profile. Therefore, the charge variation of a protein may serve as a factor for confirming its stability. $- % Acidic = \left\{area of Acidic/area of \left(Acidic+Main+Basic\right)\right\} \times 100$

For stability testing, each formulation sample was stored in a chamber maintained under accelerated stability conditions (25±2 °C and 60±5 % RH).

### 6. Analysis of oxidation level

The mass of methionine (Met₂₅₇), the 257th amino acid residue of the protein, was measured using a liquid chromatography-mass spectrometry (LC-MS) system (Waters). Following LC-MS analysis, the oxidation level (%Met₂₅₇) of methionine, the 257th amino acid residue, was determined using MassLynx^{™} and BioPharmaLynx^{™} software. 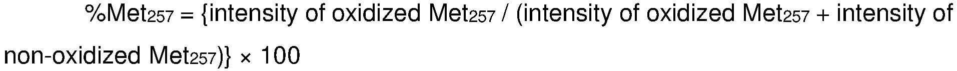 %Met257 = {intensity of oxidized Met257 / (intensity of oxidized Met257 + intensity of non-oxidized Met257)} × 100

For stability testing, each formulation sample was stored in a chamber maintained under accelerated stability conditions (25±2 °C and 60±5 %RH).

### [Examples]

### Example 1. Evaluation of T_{agg} Depending on the Presence or Absence of Methionine

Aqueous liquid formulations having the compositions shown in Table 1 below were prepared using dupilumab (CAS No. 1190264-60-8) as an anti-IL-4Rα antibody. The aggregation temperature (T_{agg}) of each formulation was measured.

An increase in T_{agg} indicates that the temperature at which aggregation occurs has increased. Therefore, an increased T_{agg} indicates improved thermal stability.

**[Table 1]**

| No.^{a} | Buffer | pH | Excipient | T_{agg} (°C) |
|---|---|---|---|---|
| 1 | 20 mM histidine | 6.0 | 8% (w/v) sucrose | 68.51 |
| 2 | | | 9% (w/v) trehalose | 69.41 |
| 3 | | | 4.7% (w/v) sorbitol | 69.54 |
| 4 | | | 3.13% (w/v) mannitol | 68.62 |
| 5 | | | 140 mM arginine | 66.76 |
| 6 | | | 135 mM lysine | 67.61 |
| 7 | | | 70 mM histidine | 67.36 |
| 8 | | | 90 mM methionine | 67.44 |
| 9 | | | 250 mM glycine | 68.32 |
| 10 | | | 0.8% (w/v) NaCl | 67.44 |
| 11^{b} | 12.5 mM acetate + 20 mM histidine | 5.9 | 5% (w/v) sucrose + 50 mM arginine | 67.89 |

| | | | | |
|---|---|---|---|---|
| ^{a} In all formulations, the antibody concentration was set to 5 mg/mL according to the required concentration for the T_{agg} analysis method. ^{b} Formulation 11 has a composition identical to that of Dupixent in terms of the buffer, pH, saccharide, and amino acid conditions. | | | | |

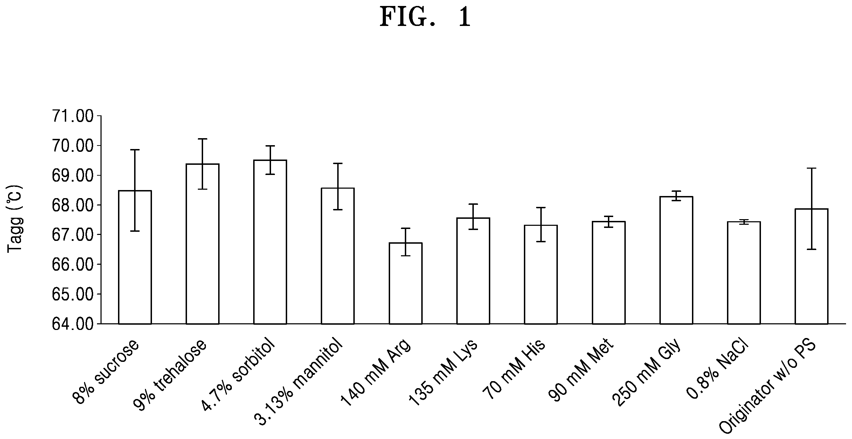

FIG. 1 is a graph showing the results of measuring an aggregation temperature (T_{agg}) for 10 formulations having different types of excipients, and a formulation having a Dupixent composition.

As shown in Table 1 and FIG. 1, Formulation 8, containing methionine, exhibited a higher aggregation temperature (T_{agg}) compared to Formulations 5 and 7, containing other types of amino acids, namely arginine or histidine.

Therefore, Formulation 8, containing methionine, demonstrated improved thermal stability attributable to an increased T_{agg}.

### Example 2. Evaluation of K_{D} Depending on the Presence or Absence of Methionine

Table 2 below presents the measured K_{D} values for the same formulations as in Example 1. Intermolecular attractive forces are a major cause of protein aggregation. An increase in K_{D} indicates a reduction in intermolecular attractive forces. Therefore, an increased K_{D} indicates improved stability.

**[Table 2]**

| No.^{a} | Buffer | pH | Excipient | K_{D} (mL/g) |
|---|---|---|---|---|
| 1 | 20 mM histidine | 6.0 | 8% (w/v) sucrose | -20 |
| 2 | | | 9% (w/v) trehalose | -14 |
| 3 | | | 4.7% (w/v) sorbitol | -6 |
| 4 | | | 3.13% (w/v) mannitol | -5 |
| 5 | | | 140 mM arginine | -3 |
| 6 | | | 135 mM lysine | -6 |
| 7 | | | 70 mM histidine | -7 |
| 8 | | | 90 mM methionine | -1 |
| 9 | | | 250 mM glycine | 0 |
| 10 | | | 0.8% (w/v) NaCl | -6 |
| 11^{b} | 12.5 mM acetate + 20 mM histidine | 5.9 | 5% (w/v) sucrose + 50 mM arginine | -9 |

| | | | | |
|---|---|---|---|---|
| ^{a} In all formulations, the antibody concentration was set to 2, 4, 6, 8, or 10 mg/mL according to the required concentration for the K_{D} analysis method. ^{b} Formulation 11 has a composition identical to that of Dupixent in terms of the buffer, pH, saccharide, and amino acid conditions. | | | | |

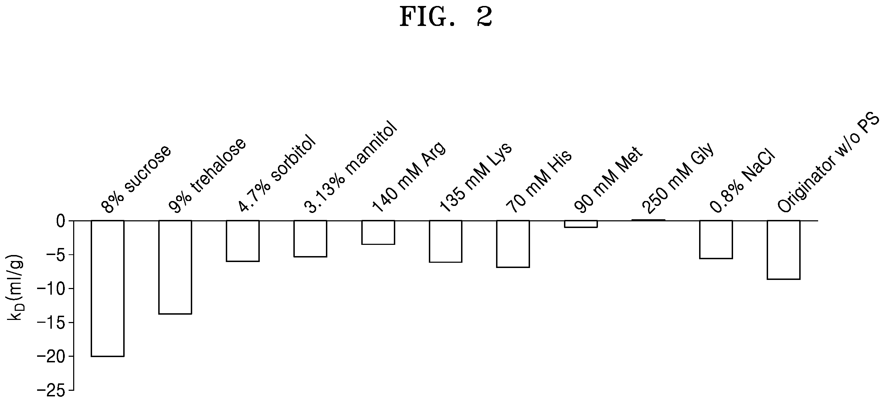

FIG. 2 is a graph showing the results of measuring a diffusion interaction parameter (K_{D}) for 10 formulations having different types of excipients, and a formulation having a Dupixent composition.

As shown in Table 2 and FIG. 2, Formulation 8, containing methionine, demonstrated an increased K_{D} compared to Formulations 1 to 7 and 10, containing sucrose, trehalose, sorbitol, mannitol, arginine, lysine, histidine, or NaCl. Furthermore, Formulation 8, containing methionine, demonstrated a significantly increased K_{D} even compared to Formulation 11 having the composition of Dupixent.

Accordingly, Formulation 8, containing methionine, demonstrated excellent stability attributable to a reduction in intermolecular attractive forces, which are a major cause of protein aggregation.

Example 3. Evaluation of Viscosity and %HMW Depending on the Presence or Absence of Methionine

Aqueous liquid formulations having the compositions shown in Table 3 below were prepared. For each formulation, the viscosity under a temperature condition of 25 °C was measured, and the %HMW under accelerated stability conditions (25 °C, 4 weeks) or stress stability conditions (40 °C, 4 weeks) was measured.

Formulations 9', 10', and 11' are formulations in which methionine is additionally included in saccharide-containing Formulations 9, 10, and 11.

Formulations 12' and 13' are formulations in which lysine is replaced with methionine in Formulations 12 and 13 containing saccharide and lysine.

**[Table 3]**

| No.^{a} | Classification | Excipient 1 | Excipient 2 | Excipient 3 | Viscosity (cP) | %HMW (25 °C, 4 weeks) | %HMW (40 °C, 4 weeks) |
|---|---|---|---|---|---|---|---|
| 9 | | 8% (w/v) trehalose | | | 33.9 | 4.27 | 7.18 |
| 9' | | 8% (w/v) trehalose | **30 mM methionine** | | 23.7 | 3.36 | 6.01 |
| 10 | Addition of methionine | 4.3% (w/v) trehalose | 80 mM histidine | | 16.8 | 2.59 | 4.42 |
| 10' | | 4.3% (w/v) trehalose | 80 mM histidine | **30 mM methionine** | 12.4 | 2.33 | 4.01 |
| 11 | | 4.3% (w/v) trehalose | 70 mM arginine | | 12.7 | 3.14 | 5.20 |
| 11' | | 4.3% (w/v) trehalose | 70 mM arginine | **30 mM methionine** | 11.5 | 2.71 | 4.63 |
| 12 | Replacement of lysine with methionine | 4.3% (w/v) trehalose | 120 mM glycine | 30 mM lysine | 31.4 | 3.68 | 6.88 |
| 12' | | 4.3% (w/v) trehalose | 120 mM glycine | **30 mM methionine** | 27.6 | 3.25 | 5.86 |
| 13 | | 1.8% (w/v) mannitol | 80 mM histidine | 30 mM lysine | 15.3 | 2.49 | 4.32 |
| 13' | | 1.8% (w/v) mannitol | 80 mM histidine | **30 mM methionine** | 13.4 | 2.35 | 4.05 |

| | | | | | | | |
|---|---|---|---|---|---|---|---|
| ^{a} All formulations contain 175 mg/mL dupilumab, 10 mM sodium acetate, and 0.2% (w/v) polysorbate 80, and have a pH of 6.0. | | | | | | | |

As shown in Table 3, the results of Formulations 9', 10', and 11' demonstrated that the addition of methionine to the saccharide-containing formulations resulted in concurrent improvements in viscosity, %HMW_{25 °C/4 weeks}, and %HMW_{40 °C/4 weeks}. These results demonstrate the excellent functionality of methionine as a viscosity-reducing agent and a stabilizer.

Furthermore, the results of Formulations 12' and 13' demonstrated that replacing lysine with methionine in the formulations containing saccharide and lysine resulted in concurrent improvements in viscosity, %HMW_{25 °C/4 weeks}, and %HMW_{40 °C/4 weeks}. These results demonstrate the superior functionality of methionine as a viscosity-reducing agent and a stabilizer compared to lysine.

### Example 4. Evaluation of %HMW, %Acidic, and %Met₂₅₇ Depending on the Presence or Absence of Methionine

Aqueous liquid formulations having the compositions shown in Table 4 below were prepared. The %HMW, %Acidic, and %Met₂₅₇ of each formulation under accelerated stability conditions (25 °C, 4 weeks) were measured.

Formulation 14' was obtained by excluding histidine from Formulation 14 containing saccharide and amino acids (i.e., histidine and methionine).

Formulation 14" was obtained by excluding methionine from Formulation 14 containing saccharide and amino acids (i.e., histidine and methionine).

**[Table 4]**

| No.^{a} | Excipient 1 | Excipient 2 | Excipient 3 | %HMW (25 °C/4 weeks) | %Acidic (25 °C/4 weeks) | %Met₂₅₇ (25 °C/4 weeks) |
|---|---|---|---|---|---|---|
| 14 | 4% (w/v) sucrose | 70 mM histidine | 25 mM methionine | 2.26 | 38.5 | 4.6 |
| 14' | 4% (w/v) sucrose | **Histidine-free** | 25 mM methionine | 3.10 | 39.7 | 5.4 |
| 14" | 4% (w/v) sucrose | 70 mM histidine | **Methionine-free** | 2.56 | 41.6 | 12.5 |

| | | | | | | |
|---|---|---|---|---|---|---|
| ^{a} All formulations contain 175 mg/mL dupilumab, 15 mM sodium acetate, and 0.2% (w/v) polysorbate 80, and have a pH of 5.5. | | | | | | |

As shown in Table 4, the results of Formulation 14" demonstrated that the exclusion of methionine from the formulation containing saccharide and amino acids resulted in concurrent increases in %HMW, %Acidic, and %Met₂₅₇ under accelerated stability conditions (25 °C, 4 weeks). In particular, the formulation excluding methionine (Formulation 14") exhibited greater increases in %Acidic and %Met₂₅₇ compared to the formulation excluding histidine (Formulation 14'). These results demonstrate the functionality of methionine as a stabilizer and an antioxidant.

Example 5. Evaluation of Viscosity and %HMW of Methionine-Containing Formulations and Methionine-Free Formulations

Aqueous liquid formulations having the compositions shown in Table 5 below were prepared. For each formulation, the viscosity at 25 °C and the %HMW immediately after preparation (Day 0, Initial) were measured.

Formulations 15 to 20 are methionine-containing formulations with varying histidine concentrations and pH values.

Formulation 21 is a methionine-free formulation having a composition identical to that of Dupixent.

**[Table 5]**

| No.^{a} | Buffer | pH | Excipient 1 | Excipient 2 | Excipient 3 | Viscosity (cP) | %HMW (Initial) |
|---|---|---|---|---|---|---|---|
| 15 | 30 mM sodium acetate | 4.9 | 4% (w/v) sucrose | 20 mM histidine | 25 mM methionine | 8.7 | 1.93 |
| 16 | | 4.8 | 4% (w/v) sucrose | 50 mM histidine | 25 mM methionine | 8.3 | 1.94 |
| 17 | | 5.2 | 4% (w/v) sucrose | 50 mM histidine | 25 mM methionine | 9.0 | 1.97 |
| 18 | | 4.9 | 4% (w/v) sucrose | 80 mM histidine | 25 mM methionine | 7.6 | 1.95 |
| 19 | | 5.5 | 4% (w/v) sucrose | 80 mM histidine | 25 mM methionine | 8.7 | 2.01 |
| 20 | | 5.2 | 4% (w/v) sucrose | 92.4 mM histidine | 25 mM methionine | 8.8 | 1.93 |
| 21^{b} | 12.5 mM sodium acetate + 20 mM histidine | 5.9 | 5% (w/v) sucrose | 50 mM arginine | - | 11.1 | 2.03 |

| | | | | | | | |
|---|---|---|---|---|---|---|---|
| ^{a} All formulations contain 175 mg/mL dupilumab and 0.2% (w/v) polysorbate 80. ^{b} Formulation 21 is the Dupixent formulation. | | | | | | | |

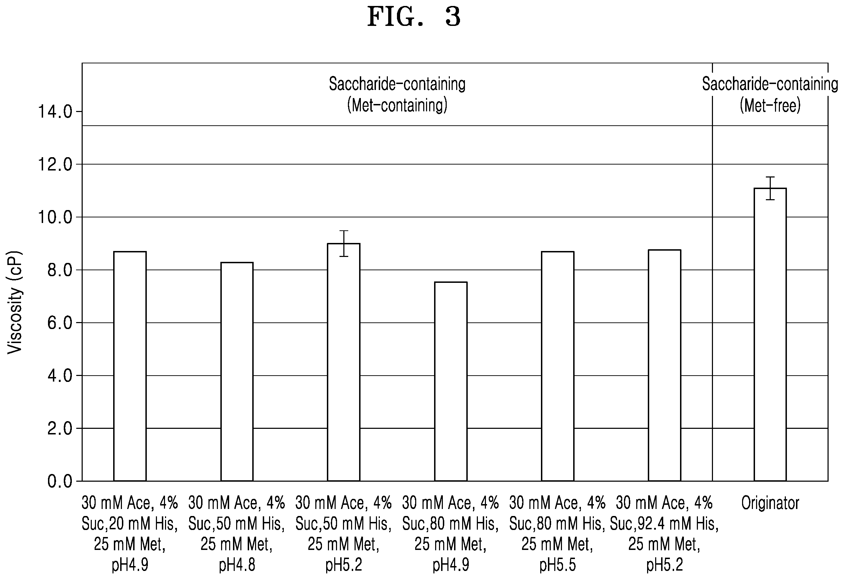

FIG. 3 is a graph showing the results of measuring a viscosity for a methionine-containing formulation and a methionine-free formulation.

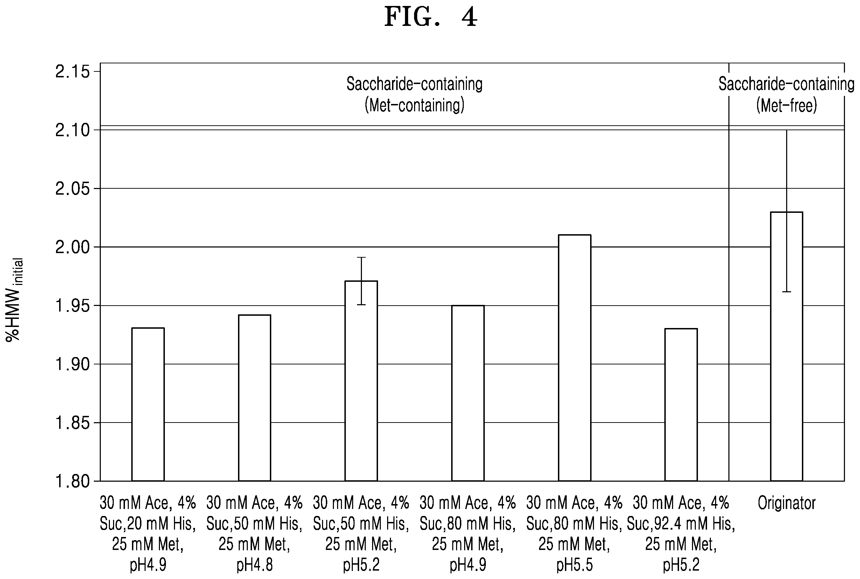

FIG. 4 is a graph showing the results of analyzing a high molecular weight species content (%HMW) for a methionine-containing formulation and a methionine-free formulation.

As shown in Table 5 and FIGS. 3 and 4, the 25 mM methionine-containing formulations (Formulations 15 to 20), containing 20 to 92.4 mM histidine and having a pH ranging from 4.9 to 5.5, demonstrated concurrent improvements in viscosity and %HMW compared to the methionine-free Dupixent formulation (Formulation 21).

The above description of the present disclosure is provided for the purpose of illustration, and those of ordinary skill in the art to which the present disclosure pertains will understand that the present disclosure may be easily modified into other specific forms without changing the technical spirit or essential features thereof. Therefore, it should be understood that the embodiments described above are illustrative in all respects and not restrictive.

## Claims

1. A liquid formulation comprising:
an anti-IL-4Rα antibody;
a buffer;
a saccharide; and
methionine,
wherein the liquid formulation has a pH of about 4.9 to about 5.5.

2. The liquid formulation of claim 1, wherein the anti-IL-4Rα antibody is dupilumab.

3. The liquid formulation of claim 1, wherein a concentration of the anti-IL-4Rα antibody is about 5 mg/mL to about 300 mg/mL.

4. The liquid formulation of claim 1, wherein a concentration of the anti-IL-4Rα antibody is about 100 mg/mL to about 200 mg/mL.

5. The liquid formulation of claim 1, wherein a concentration of the anti-IL-4Rα antibody is about 125 mg/mL to about 200 mg/mL.

6. The liquid formulation of claim 1, wherein the buffer comprises acetate.

7. The liquid formulation of claim 1, wherein the buffer is sodium acetate.

8. The liquid formulation of any one of claims 1 to 7, wherein a concentration of the buffer is about 10 mM to about 50 mM.

9. The liquid formulation of claim 6, wherein a concentration of the acetate is about 10 mM to about 40 mM.

10. The liquid formulation of claim 6, wherein a concentration of the acetate is about 15 mM to about 35 mM.

11. The liquid formulation of claim 1, wherein the saccharide comprises one or more selected from a sugar and a sugar alcohol.

12. The liquid formulation of claim 11, wherein the sugar comprises one or more selected from sucrose, trehalose, galactose, mannose, maltose, lactose, fructose, and glucose.

13. The liquid formulation of claim 11, wherein the sugar alcohol comprises one or more selected from mannitol, sorbitol, xylitol, arabitol, erythritol, lactitol, maltitol, and inositol.

14. The liquid formulation of claim 11, wherein the saccharide comprises one or more selected from sucrose, trehalose, and mannitol.

15. The liquid formulation of claim 11, wherein the saccharide is sucrose.

16. The liquid formulation of claim 11, wherein a concentration of the sugar is about 1% (w/v) to about 20% (w/v).

17. The liquid formulation of claim 11, wherein a concentration of the sugar alcohol is about 1% (w/v) to about 20% (w/v).

18. The liquid formulation of claim 15, wherein a concentration of the sucrose is about 1% (w/v) to about 20% (w/v).

19. The liquid formulation of claim 1, wherein a concentration of the methionine is about 2.7 mM to about 90 mM.

20. The liquid formulation of claim 1, wherein a concentration of the methionine is about 20 mM to about 30 mM.

21. The liquid formulation of claim 1, wherein the methionine has one or more functions of a viscosity reducing agent, a stabilizer, and an antioxidant.

22. The liquid formulation of any one of claims 1 to 21, further comprising histidine.

23. The liquid formulation of claim 12, wherein a concentration of the histidine is about 20 mM to about 92.4 mM.

24. The liquid formulation of claim 12, wherein a concentration of the histidine is about 30 mM to about 92.4 mM.

25. The liquid formulation of any one of claims 1 to 24, wherein the liquid formulation is free of arginine.

26. The liquid formulation of any one of claims 1 to 25, further comprising a surfactant.

27. The liquid formulation of claim 26, wherein the surfactant comprises a nonionic surfactant.

28. The liquid formulation of claim 26, wherein the surfactant comprises polysorbate 20, polysorbate 80, or a combination thereof.

29. The liquid formulation of any one of claims 26 to 28, wherein a concentration of the surfactant is 0.01% (w/v) to 0.9% (w/v).

30. The liquid formulation of any one of claims 26 to 28, wherein a concentration of the surfactant is about 0.1% (w/v) to about 0.3% (w/v).

31. The liquid formulation of any one of claims 1 to 30, wherein a viscosity of the liquid formulation is 5.0 cP to 11.0 cP.

32. The liquid formulation of any one of claims 1 to 30, wherein:
a high molecular weight species content (%HMW) of the antibody measured after storage at 25 °C for 4 weeks is 3.1 % or less;
an acidic species content (%Acidic) of the antibody measured after storage at 25 °C for 4 weeks is less than 41 %; or
an oxidation rate (%Met₂₅₇) of a methionine residue in the antibody measured after storage at 25 °C for 4 weeks is less than 12 %.

33. The liquid formulation of any one of claims 1 to 30, wherein a high molecular weight species content (%HMW) of the antibody measured immediately after preparation is less than 2.03 %.

34. A liquid formulation comprising:
about 5 mg/mL to about 300 mg/mL of dupilumab;
about 10 mM to about 40 mM of acetate;
about 10 mM to about 100 mM of methionine;
about 20 mM to about 92.4 mM of histidine;
about 2% (w/v) to about 10% (w/v) of sucrose; and
about 0.1% (w/v) to about 0.9% (w/v) of polysorbate 80,
wherein the liquid formulation has a pH of about 4.9 to about 5.5.

35. A liquid formulation comprising:
100 mg/mL to 200 mg/mL of dupilumab;
30 mM±5 mM of acetate;
25 mM±5 mM of methionine;
20 mM to 92.4 mM of histidine;
4%±1.0% (w/v) of sucrose; and
0.2%±0.1% (w/v) of polysorbate 80,
wherein the liquid formulation has a pH of about 4.9 to about 5.5.

36. A liquid formulation comprising:
100 mg/mL to 200 mg/mL of dupilumab;
30 mM±5 mM of acetate;
25 mM±5 mM of methionine;
20 mM to 92.4 mM of histidine;
5.5%±1.0% (w/v) of sucrose; and
0.2%±0.1% (w/v) of polysorbate 80,
wherein the liquid formulation has a pH of about 4.9 to about 5.5.

37. The liquid formulation of any one of claims 1 to 36, wherein the liquid formulation is for subcutaneous injection.

38. A device comprising the liquid formulation of any one of claims 1 to 37.

39. The device of claim 38, comprising the liquid formulation in a container selected from a syringe, a pre-filled syringe, a pre-filled pen, a microinfuser, an autoinjector, a bottle, a vial, and a tube.

40. A method of treating an IL-4Rα-related condition, comprising administering the liquid formulation of any one of claims 1 to 37 to a subject in need thereof.

41. The method of claim 40, wherein the IL-4Rα-related condition is an IL-4-mediated disease or an IL-13-mediated disease.

42. The method of claim 40, wherein the IL-4Rα-related condition is any one selected from atopic dermatitis, asthma, chronic rhinosinusitis (CRS), chronic rhinosinusitis with nasal polyposis (CRSwNP), allergic fungal rhinosinusitis (AFRS), eosinophilic esophagitis (EoE), prurigo nodularis (PN), bullous pemphigoid (BP), chronic spontaneous urticaria (CSU), chronic inducible urticaria (CIndU), and chronic obstructive pulmonary disease (COPD).

43. Use of the liquid formulation of any one of claims 1 to 37 in the manufacture of a medicament for treating an IL-4Rα-related condition.
